# EUROPÄISCHE PATENTSCHRIFT

(11) **EP 2 626 845 B1**
(45) Veröffentlichungstag und Bekanntmachung des Hinweises auf die Patenterteilung: **09.03.2016**
(21) Anmeldenummer: 12198815.8
(22) Anmeldetag: 21.12.2012
(51) Int. Cl.: G08B 21/04, A61B 5/00

(54) **Verfahren und Sensor-System zur sensorischen Raum-Überwachung**
Method and sensor system for the sensory monitoring of rooms
Procédé et système de capteur pour la surveillance d'espace sensorielle

(30) Priorität: 09.02.2012 DE 102012101062
(43) Veröffentlichungstag der Anmeldung: 14.08.2013
(73) Patentinhaber: GIRA GIERSIEPEN GmbH & Co. KG, 42477 Radevormwald (DE)
(72) Erfinder: Joska, Rolf, 46119 Oberhausen (DE)
(74) Vertreter: Patentanwälte Dr. Solf & Zapf

(56) Entgegenhaltungen:
- EP-A1- 1 160 751
- WO-A1-2007/101343
- US-A1- 2008 294 019
- CHIOUKH L ET AL: "Integrated radar systems for precision monitoring of heartbeat and respiratory status", MICROWAVE CONFERENCE, 2009. APMC 2009. ASIA PACIFIC, IEEE, PISCATAWAY, NJ, USA, 7. Dezember 2009 (2009-12-07), Seiten 405-408, XP031613448, ISBN: 978-1-4244-2801-4
- B J. Jang ET AL: "WIRELESS BIO-RADAR SENSOR FOR HEARTBEAT AND RESPIRATION DETECTION", Progress In Electromagnetics Research C,, 1. Januar 2008 (2008-01-01), Seiten 149-168, XP055060341, Gefunden im Internet: URL:http://www.jpier.org/PIERC/pierc05/14. 08110603.pdf [gefunden am 2013-04-19]
- "BioRadar BR 402 brief description", , 1. Mai 2011 (2011-05-01), XP055060399, Gefunden im Internet: URL:http://www.bos-berlin.de/fileadmin/use r_upload/docs/products_en/BioRadar402_brie f_descrip.pdf [gefunden am 2013-04-19]
- QIANG WANG ET AL: "Life signal extraction in through-the-wall surveillance", PROCEEDINGS OF THE 31ST ANNUAL INTERNATIONAL CONFERENCE OF THE IEEE ENGINEERING IN MEDICINE AND BIOLOGY SOCIETY: ENGINEERING THE FUTURE OF BIOMEDICINE, EMBC 2009, IEEE, 3. September 2009 (2009-09-03), Seiten 1343-1346, XP031882192, DOI: 10.1109/IEMBS.2009.5334151 ISBN: 978-1-4244-3296-7
- JENSHAN LIN ET AL: "Wireless Non-Contact Detection of Heartbeat and Respiration Using Low-Power Microwave Radar Sensor", MICROWAVE CONFERENCE, 2007. APMC 2007. ASIA-PACIFIC, IEEE, PISCATAWAY, NJ, USA, 11. Dezember 2007 (2007-12-11), Seiten 1-4, XP031279794, ISBN: 978-1-4244-0748-4
- QUIRIN HAMP ET AL: "Lessons learned from german research for USAR", SAFETY, SECURITY, AND RESCUE ROBOTICS (SSRR), 2011 IEEE INTERNATIONAL SYMPOSIUM ON, IEEE, 1. November 2011 (2011-11-01), Seiten 26-31, XP032076639, DOI: 10.1109/SSRR.2011.6106758 ISBN: 978-1-61284-770-2

## Beschreibung

Die vorliegende Erfindung betrifft zunächst gemäß dem unabhängigen Anspruch 1 ein Verfahren zur sensorischen Überwachung eines Raumes. Ferner betrifft die Erfindung gemäß dem Anspruch 3 auch ein entsprechendes Sensor-System.

Es ist bekannt, Räume mit Bewegungssensoren, häufig auch Bewegungsmelder genannt, zu überwachen, um Personen zu erfassen und dann bestimmte Maßnahmen auszulösen. Beispielsweise kann automatisch Licht eingeschaltet und/oder ein Alarm ausgelöst werden, wenn eine Person den Raum betritt.

Der vorliegenden Erfindung liegt die Aufgabe zu Grunde, ein Verfahren anzugeben und ein Sensor-System zu schaffen, womit zusätzliche Nutzungsmöglichkeiten und Anwendungsvorteile erreicht werden.

Diese Aufgabe wird durch ein Verfahren nach dem Anspruch 1 einerseits und durch ein Sensor-system nach dem Anspruch 3 andererseits gelöst. Vorteilhafte Ausgestaltungen und besondere Maßnahmen der Erfindung sind in den jeweils abhängigen Ansprüchen enthalten.

Erfindungsgemäß wird demnach der Raum mit einem Bioradar-Sensor überwacht, um bei Anwesenheit mindestens einer Person dessen so genannte Vitalparameter zu erfassen, und die erfassten Vitalparameter werden mit gespeicherten Normalparametern verglichen, wobei im Falle von bestimmten Abweichungen ein Signal, insbesondere ein Notruf ausgelöst wird.

Durch die Erfindung ist es vorteilhafterweise möglich, Personen zu erfassen und "drahtlos", ohne Leitungsverbindungen bezüglich ihrer Vitalparameter zu überwachen, und im Falle von Abweichungen, d. h. bei Störungen der "normalen" Körperfunktionen, z. B. ein Alarmsignal auszulösen. Übliche Vitalparameter sind Herzschlag (Herzbewegungen) und/oder Atembewegungen. So ist es möglich, eine z. B. in einem Bett liegende und gegebenenfalls schlafende Person zu überwachen und bei kritischen Änderungen oder gar einem Stillstand der Herz- und/oder Atembewegungen ein Notrufsignal auszulösen. Dies stellt praktisch eine automatische Vitalfunktionsüberwachung dar. Die Erfindung eignet sich deshalb besonders für Anwendungen in Krankenhäusern, Alten- und Pflegeeinrichtungen, Kinder- und Säuglingszimmern sowie aber auch für so genannte barrierefreie Wohnbereiche insbesondere für ältere Personen und für Menschen mit Behinderungen. Damit bildet das erfindungsgemäße Sensor-System ein "Altersgerechtes Assistenzsystem für ein gesundes und unabhängiges Leben", kurz "AAL", Stichwort "Ambient Assisted Living".

Dokument WO2007101343 stellt den nächstliegenden Stand der Technik dar und offenbart die Überwachung eines Raumes mittels Bioradar-Sensoren zur Erfassung von Vitalparametern. Diese Bioradar-Sensoren können zwecks des Erfassens der Präsenz oder der Aktivität einer Person mit anderen Sensoren kombiniert werden.

Anhand der beigefügten Zeichnungen soll die Erfindung im Folgenden beispielhaft genauer erläutert werden. Es zeigen:
- Fig. 1: ein vereinfachtes, schematisches Blockschaltbild eines erfindungsgemäßen Sensor-Systems und
- Fig. 2: ein Ausführungsbeispiel einer Sensoreinheit für das Sensor-System nach Fig. 1.

In den verschiedenen Figuren der Zeichnung sind gleiche Teile stets mit den gleichen Bezugszeichen versehen.

Zu der anschließenden Beschreibung wird ausdrücklich betont, dass die Erfindung nicht auf die Ausführungsbeispiele und dabei nicht auf alle oder mehrere Merkmale von beschriebenen Merkmalskombinationen beschränkt ist, vielmehr kann jedes einzelne Teilmerkmal des/jedes Ausführungsbeispiels auch losgelöst von allen anderen im Zusammenhang damit beschriebenen Teilmerkmalen für sich und auch in Kombination mit beliebigen Merkmalen eines anderen Ausführungsbeispiels sowie auch unabhängig von den Merkmalskombinationen und Rückbeziehungen der Ansprüche eine erfinderische Bedeutung haben.

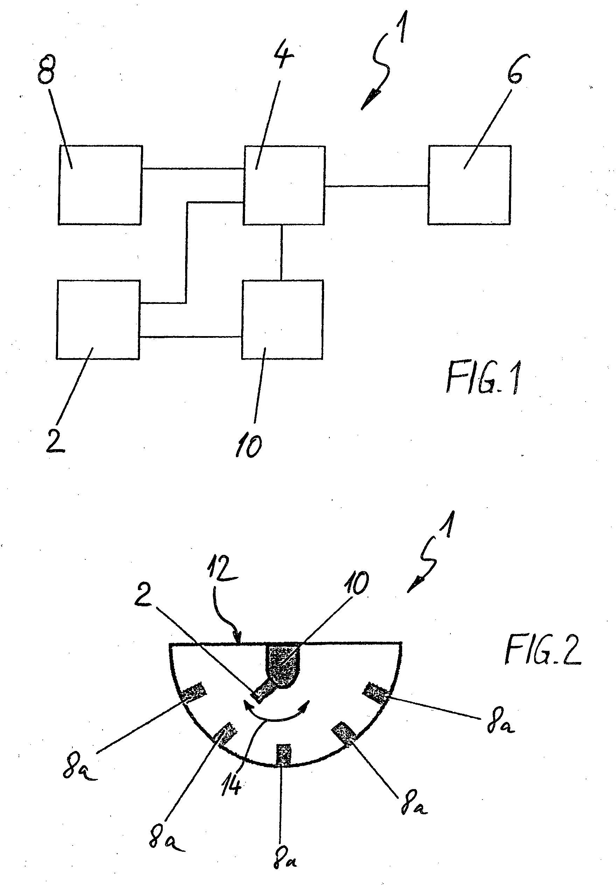

Gemäß Fig. 1 weist ein erfindungsgemäßes Sensor-System 1 einen Bioradar-Sensor 2 zur Erfassung von bestimmten Vitalparametern einer in einem Raum befindlichen Person auf. Der Bioradar-Sensor 2 ist mit einer elektronischen Auswerteeinrichtung 4 verbunden, die derart ausgelegt ist, dass sie jeweils über den Biorädar-Sensor 2 erfasste Vitalparameter mit vorgegebenen, gespeicherten Normalparametern vergleicht und im Falle von bestimmten Abweichungen der Vitalparameter von den Normalparametern über eine Signaleinrichtung 6 ein Signal, insbesondere einen Notruf auslöst.

Das erfindungsgemäße Sensor-System 1 weist zusätzlich einen Bewegungssensor 8 zum Erfassen der Anwesenheit und der Position einer Person innerhalb des Raumes auf. In Verbindung damit ist ein Steuerteil 10 zum mechanischen Ausrichten des Bioradar-Sensors 2 auf die über den Bewegungssensor 8 erfasste Position der Person vorgesehen. Dazu wertet die Auswerteeinrichtung 4 die Signale des Bewegungssensors 8 aus und steuert das Steuerteil 10 so an, dass mittels des Steuerteils 10 der Bioradar-Sensor 2 selbsttätig auf die Position der Person ausgerichtet wird.

Wie sich aus Fig. 2 ergibt, können die besagten Sensoren gemeinsam in einer Sensoreinheit 12 angeordnet sein, wobei der Bewegungssensor 8 aus mehreren Einzelsensoren 8a bestehen kann, die zur Überwachung eines Raum-Winkelbereiches über einen Kreisumfang derart verteilt angeordnet sind, dass ein Raum-Winkelbereich von vorzugsweise mindestens 180° überwacht werden kann. Der Bioradar-Sensor 2 ist über das Steuerteil 10 über den Raum-Winkelbereich hinweg schwenkbar angeordnet, so dass er in eine bestimmte Winkelausrichtung bewegt werden kann, was durch einen Doppelpfeil 14 angedeutet ist.

Es ist vorteilhaft, den Bioradar-Sensor 2 erst nach Erfassung einer Person zu aktivieren. Grundsätzlich können dann die Vitalparameter permanent erfasst werden, allerdings ist es ausreichend, eine Erfassung und Auswertung nur in bestimmten Zeitintervallen durchzuführen.

Die Erfindung ist nicht auf die dargestellten und beschriebenen Ausführungsbeispiele beschränkt, sondern umfasst auch alle im Sinne der Erfindung gleichwirkenden Ausführungen. Es wird ausdrücklich betont, dass die Ausführungsbeispiele nicht auf alle Merkmale in Kombination beschränkt sind, vielmehr kann jedes einzelne Teilmerkmal auch losgelöst von allen anderen Teilmerkmalen für sich eine erfinderische Bedeutung haben.

Die Erfindung wird durch die anbängigen Ansprüche definiert.

## Patentansprüche

1. Verfahren zur sensorischen Überwachung eines Raumes,
wobei der Raum bei Anwesenheit mindestens einer Person mit einem Bioradar-Sensor (2) zur Erfassung von Vitalparametern überwacht wird, wobei erfasste Vitalparameter mit gespeicherten Normalparametern verglichen werden und bei bestimmten Abweichungen ein Signal ausgelöst wird,
**dadurch gekennzeichnet, dass** der Raum zunächst mit einem Bewegungssensor (8) auf Anwesenheit einer Person und auf die Position der Person innerhalb des Raumes überwacht wird, wobei dann der Bioradar-Sensor (2) auf die erfasste Position der Person ausgerichtet und aktiviert wird.

2. Verfahren nach Anspruch 1,
**dadurch gekennzeichnet, dass** nach Aktivierung des Bioradar-Sensors(2) die Vitalparameter permanent, vorzugsweise aber nur in bestimmten Zeitintervallen erfasst und ausgewertet werden.

3. Sensor-System (1) zur Überwachung eines Raumes, insbesondere zur Durchführung des Verfahrens nach Anspruch 1 oder 2, umfassend einen Bioradar-Sensor (2) zur Erfassung von Vitalparametern einer in dem Raum befindlichen Person, eine Auswerteeinrichtung (4) zum Vergleichen der erfassten Vitalparameter mit vorgegebenen, gespeicherten Normalparametern sowie eine Signaleinrichtung (6) zur Auslösung eines Signals im Falle von bestimmten Abweichungen der Vitalparameter von den Normalparametern, **gekennzeichnet durch** einen Bewegungssensor (8) zum Erfassen der Anwesenheit und der Position einer Person innerhalb des Raumes und ein Steuerteil (10) zum Ausrichten des Bioradar-Sensors (2) auf die erfasste Position der Person.

4. Sensor-System nach Anspruch 3,
**dadurch gekennzeichnet, dass** der Bewegungssensor (8) mehrere Einzelsensoren (8a) zur Überwachung eines Raum-Winkelbereiches über insbesondere mindestens 180° aufweist.

## Claims

1. Method for monitoring a space by sensors,
the space being monitored by a bio-radar sensor (2) for registering vital parameters when at least one person is present, with registered vital parameters being compared to stored normal parameters and a signal being triggered in the case of specific deviations,
**characterized in that** the space is initially monitored by a motion sensor (8) for the presence of a person and for the position of the person within the space, with the bio-radar sensor (2) then being directed to the registered position of the person and activated.

2. Method according to Claim 1,
**characterized in that** the vital parameters are registered and evaluated permanently, but preferably only at specific time intervals, after activating the bio-radar sensor (2).

3. Sensor system (1) for monitoring a space, in particular for carrying out the method according to Claim 1 or 2, comprising a bio-radar sensor (2) for registering vital parameters of a person situated in the space, an evaluation apparatus (4) for comparing the registered vital parameters with predetermined, stored normal parameters, and a signal apparatus (6) for triggering a signal in the case of specific deviations of the vital parameters from the normal parameters,
**characterized by** a motion sensor (8) for registering the presence and the position of a person within the space and a control part (10) for aligning the bio-radar sensor (2) onto the registered position of the person.

4. Sensor system according to Claim 3,
**characterized in that** the motion sensor (8) has a plurality of individual sensors (8a) for monitoring a solid angle range over, in particular, at least 180°.

## Revendications

1. Procédé de surveillance par capteurs d'un espace,
dans lequel l'espace est surveillé lors de la présence d'au moins une personne munie d'un capteur à bio-radar (2) pour détecter des paramètres vitaux, dans lequel les paramètres vitaux détectés sont comparés à des paramètres normaux stockés en mémoire et un signal est généré lorsque des écarts sont déterminés,
**caractérisé en ce que** l'espace est tout d'abord surveillé au moyen d'un capteur de mouvement (8) en ce qui concerne la présence d'une personne et la position de la personne à l'intérieur de l'espace, dans lequel le capteur à bio-radar (2) est ensuite orienté et activé vers la position détectée de la personne.

2. Procédé selon la revendication 1,
**caractérisé en ce que**, après activation du capteur à bio-radar (2), les paramètres vitaux sont détectés et évalués en permanence mais de préférence uniquement à des intervalles de temps déterminés.

3. Système de capteur (1) destiné à surveiller un espace, notamment destiné à mettre en oeuvre le procédé selon la revendication 1 ou 2, comprenant un capteur à bio-radar (2) destiné à détecter des paramètres vitaux d'une personne se trouvant dans l'espace, un dispositif d'évaluation (4) destiné à comparer les paramètres vitaux détectés à des paramètres normaux prédéterminés stockés en mémoire et un dispositif générateur de signal (6) destiné à générer un signal dans le cas où des écarts sont déterminés entre les paramètres vitaux et les paramètres normaux,
**caractérisé par** un capteur de mouvement (8) destiné à détecter la présence et la position d'une personne à l'intérieur de l'espace et une partie de commande (10) destinée à orienter le capteur à bio-radar (2) vers la position détectée de la personne.

4. Système de capteur selon la revendication 3,
**caractérisé en ce que** le capteur de mouvement (8) comprend de multiples capteurs individuels (8a) destiné à surveiller une zone angulaire de l'espace couvrant notamment au moins 180°.
